# EUROPEAN PATENT SPECIFICATION

(11) **EP 4 370 917 B1**
(45) Date of publication and mention of the grant of the patent: **09.12.2026**
(21) Application number: 22760829.6
(22) Date of filing: 12.07.2022
(51) Int. Cl.: G01N 30/96, G01N 33/68

(54) **CHARACTERIZATION OF PROTEINS BY ANION-EXCHANGE CHROMATOGRAPHY MASS SPECTROMETRY (AEX-MS)**
CHARAKTERISIERUNG VON PROTEINEN DURCH ANIONENAUSTAUSCHCHROMATOGRAFIE-MASSENSPEKTROMETRIE (AEX-MS)
CARACTÉRISATION DE PROTÉINES PAR SPECTROMÉTRIE DE MASSE PAR CHROMATOGRAPHIE D'ÉCHANGE D'ANIONS (AEX-MS)

(30) Priority: 13.07.2021 US 202163221447 P; 31.01.2022 US 202263305177 P
(43) Date of publication of application: 22.05.2024
(73) Proprietor: Regeneron Pharmaceuticals, Inc., Tarrytown, NY 10591 (US)
(72) Inventor: LIU, Anita, Tarrytown, New York 10591 (US); WANG, Shunhai, Tarrytown, New York 10591 (US)
(74) Representative: Carpmaels & Ransford LLP
(86) International application number: PCT/US2022/036868
(87) International publication number: WO 2023/287821

(56) References cited:
- WO-A1-2014/078729
- US-A1- 2010 219 335
- US-A1- 2019 234 964
- US-A1- 2020 225 199
- PONNIAH GOMATHINAYAGAM ET AL: "Characterization of charge variants of a monoclonal antibody using weak anion exchange chromatography at subunit levels", ANALYTICAL BIOCHEMISTRY, ACADEMIC PRESS, AMSTERDAM, NL, vol. 520, 23 December 2016 (2016-12-23), pages 49 - 57, XP029894099, ISSN: 0003-2697, DOI: 10.1016/J.AB.2016.12.017
- BIGELOW ELIZABETH ET AL: "Using continuous chromatography methodology to achieve high-productivity and high-purity enrichment of charge variants for analytical characterization", JOURNAL OF CHROMATOGRAPHY A, ELSEVIER, AMSTERDAM, NL, vol. 1643, 18 March 2021 (2021-03-18), XP086534842, ISSN: 0021-9673, [retrieved on 20210318], DOI: 10.1016/J.CHROMA.2021.462008

## Description

### CROSS-REFERENCE TO RELATED APPLICATIONS

This application claims priority to and the benefit of U.S. Provisional Patent Application No. 63/221,447, filed July 13, 2021. This application also claims priority to and the benefit of Provisional Application No. U.S. 63/305,177, filed January 31, 2022.

### FIELD

The present invention generally pertains to methods for characterizing charge variants of proteins using anion-exchange chromatography coupled to mass spectrometry using increasing salt gradient elution.

### BACKGROUND

Therapeutic proteins are important drugs for the treatment of cancer, autoimmune disease, infection and cardiometabolic disorders, and they represent one of the fastest growing product segments of the pharmaceutical industry. They must meet very high standards of purity. Thus, it can be important to monitor impurities at different stages of drug development, production, storage and handling.

More than 600 different types of post-translational modifications are known for proteins, many of them being of extremely low abundance, causing subtle changes in physicochemical properties and posing an extreme challenge to analytical methods required for their characterization. For proteins produced using recombinant methods, post-translational modifications represent critical product quality attributes, which may impact drug potency and patient safety, as has been shown for monoclonal antibodies (mAbs). Charge-sensitive separation modes such as ion exchange chromatography (IEC), capillary electrophoresis, and capillary isoelectric focusing are routinely used for the separation and characterization of charge variants, due to their high selectivity, allowing for the separation of protein variants with minimal difference in their properties, *i.e.,* net surface charge. However, charge-based separation methods for intact proteins are generally not ideally suited for coupling to mass spectrometry (MS) due to non-compatibility of the buffer systems. The methods developed so far by coupling IEC with MS have only been suitable for proteins with isoelectric point (pI) less than about 6.0.

Several therapeutic proteins developed based on IgG1 and IgG4 can have pI greater than 6.0. Development, purification, and characterization of these proteins require an efficient analytical method. It will be appreciated that there is a long felt need in the art for an efficient method for characterizing charge variants of proteins irrespective of their pIs. G. PONNIAH et al.: "Characterization of charge variants of a monoclonal antibody using weak anion exchange chromatography at subunit levels"; Analytical Biochemistry 520 (2017), pages 49-57, discloses the use of a NaCl based mobile phase for the separation of antibody charge variants.

### SUMMARY

Exemplary embodiments disclosed herein satisfy the aforementioned demands by providing methods for characterizing charge variants of a protein using native AEX-MS.

This disclosure provides a method for characterization of such charge variants of a protein of interest. The method comprises: (a) loading a sample having a protein of interest and at least one charge variant of said protein of interest to an anion-exchange chromatography (AEX) column; (b) applying an increasing salt concentration gradient to the loaded anion exchange column to obtain an eluate; (c) collecting at least one fraction from (b); and (d) subjecting said at least one fraction to mass spectrometry analysis to characterize said at least one charge variant of said protein of interest.

The method further comprises applying an increasing salt concentration gradient to the loaded anion exchange column, wherein the salt gradient is an elution gradient and ranges from about 10 mM to about 600 mM ammonium salt. In one specific aspect, the ammonium salt is ammonium acetate. In another specific aspect, the salt gradient is an elution gradient and ranges from about 10 mM to about 300 mM ammonium salt. In another specific aspect, the increasing salt concentration gradient applied is linear.

In one aspect of the embodiment, the method further comprises monitoring the eluate of (b) for ultraviolet absorbance and collecting said at least one fraction that is eluted prior to elution of the protein of interest. In another aspect of the embodiment, the method further comprises monitoring the eluate of (b) for ultraviolet absorbance and collecting said at least one fraction that is eluted after elution of the protein of interest.

The protein of interest has a pI value of about 6.2 to about 7.0.

In one aspect of the embodiment, the protein of interest is an IgG4-based monoclonal antibody. In another aspect of the embodiment, the protein of interest is an IgG1 -based monoclonal antibody. In yet another aspect of the embodiment, the protein of interest is a bispecific monoclonal antibody.

In one aspect of the embodiment, the method further comprises reducing flow rate from (c) via a flow-splitter prior to subjecting said at least one fraction to a mass spectrometer.

In one aspect of the embodiment, said at least one charge variant can be either an acidic variant or a basic variant of the protein of interest. For example, the acidic variants can include deamidation, glycation, glucuronylation, and/or high molecular weight species of the protein of interest, and the basic variants can include C-terminal lysine, and glycosylated species.

In one aspect of the embodiment, the method further comprises removing Fc N-glycosylation from said protein of interest prior to loading said sample on said AEX column. This step can improve the AEX separation due to conversion of Asparagine residues to Aspartic acid. In a specific aspect of this embodiment, the Fc N-glycosylation from said protein of interest is removed using PNGase F. In another specific aspect of this embodiment, the method can further comprise treating said sample to digestion conditions.

In some exemplary embodiments, the mass spectrometer is run under native conditions.

In one aspect of the embodiment, the method further comprises treating said sample to digestion conditions prior to loading said sample on AEX column. In one specific aspect, the digestion conditions include use of IdeS or a variant thereof.

In one exemplary embodiment, the method comprises: (a) subjecting a sample having a protein of interest and at least one charge variant to deglycosylation conditions; (b) loading said sample to an anion-exchange chromatography (AEX) column; (c) applying an increasing salt concentration gradient to the loaded anion exchange column to obtain an eluate; (d) collecting at least one fraction from (c); and (e) subjecting said at least one fraction to mass spectrometry analysis to characterize said at least one charge variant of said protein of interest.

The salt gradient is an elution gradient and ranges from about 10 mM to about 600 mM ammonium salt. In one specific aspect, the ammonium salt is ammonium acetate. In another specific aspect, the salt gradient is an elution gradient and ranges from about 10 mM to about 300 mM ammonium salt. In another specific aspect, the increasing salt concentration gradient applied is linear.

In one aspect of the embodiment, the method further comprises monitoring the eluate of (c) for ultraviolet absorbance and collecting said at least one fraction that is eluted prior to elution of the protein of interest. In another aspect of the embodiment, the method further comprises monitoring the eluate of (c) for ultraviolet absorbance and collecting said at least one fraction that is eluted after elution of the protein of interest.

The protein of interest has a pI value of about 6.2 to about 7.0.

In one aspect of the embodiment, the protein of interest is an IgG4-based monoclonal antibody. In another aspect of the embodiment, the protein of interest is an IgG1 -based monoclonal antibody. In yet another aspect of the embodiment, the protein of interest is a bispecific monoclonal antibody.

In one aspect of the embodiment, the method further comprises reducing flow rate from (d) via a flow-splitter prior to subjecting said at least one fraction to a mass spectrometer.

In one aspect of the embodiment, said at least one charge variant can be either an acidic variant or a basic variant of the protein of interest. For example, the acidic variants can include deamidation, glycation, glucuronylation, and/or high molecular weight species of the protein of interest, and the basic variants can include C-terminal lysine and glycosylated species.

In one aspect of the embodiment, the method further comprises removing Fc N-glycosylation from said protein of interest prior to loading said sample on AEX column. This step can improve the AEX separation due to conversion of Asparagine residues to Aspartic acid. In a specific aspect of this embodiment, the Fc N-glycosylation from said protein of interest is removed using PNGase F.

In some exemplary embodiments, the mass spectrometer is run under native conditions.

In one aspect of the embodiment, the method further comprises treating said sample to digestion conditions prior to loading said sample on said AEX column. In one specific aspect, the digestion conditions include use of IdeS or a variant thereof.

In one exemplary embodiment, the method comprises: (a) subjecting a sample having a protein of interest and at least one charge variant to digestion conditions and deglycosylation conditions; (b) loading said sample to an anion-exchange chromatography (AEX) column; (c) applying an increasing salt concentration gradient to the loaded anion exchange column to obtain an eluate; (d) collecting at least one fraction from (c); and (e) subjecting said at least one fraction to mass spectrometry analysis to characterize said at least one charge variant of said protein of interest.

In one aspect of the embodiment, the digestion conditions include the use of IdeS or a variant thereof.

The salt gradient is an elution gradient and ranges from about 10 mM to about 600 mM ammonium salt. In one specific aspect, the ammonium salt is ammonium acetate. In another specific aspect, the salt gradient is an elution gradient and ranges from about 10 mM to about 300 mM ammonium salt. In another specific aspect, the increasing salt concentration gradient applied is linear.

In one aspect of the embodiment, the method further comprises monitoring the eluate of (c) for ultraviolet absorbance and collecting said at least one fraction that is eluted prior to elution of the protein of interest. In another aspect of the embodiment, the method further comprises monitoring the eluate of (c) for ultraviolet absorbance and collecting said at least one fraction that is eluted after elution of the protein of interest.

The protein of interest has a pI value of about 6.2 to about 7.0.

In one aspect of the embodiment, the protein of interest is an IgG4-based monoclonal antibody. In another aspect of the embodiment, the protein of interest is an IgG1 -based monoclonal antibody. In yet another aspect of the embodiment, the protein of interest is a bispecific monoclonal antibody.

In one aspect of the embodiment, the method further comprises reducing flow rate from (d) via a flow-splitter prior to subjecting said at least one fraction to a mass spectrometer.

In one aspect of the embodiment, said at least one charge variant can be either an acidic variant or a basic variant of the protein of interest. For example, the acidic variants can include deamidation, glycation, glucuronylation, and/or high molecular weight species of the protein of interest, and the basic variants can include C-terminal lysine and glycosylated species.

In one aspect of the embodiment, the method further comprises removing Fc N-glycosylation from said protein of interest prior to loading said sample on said AEX column. This step can improve the AEX separation due to conversion of Asparagine residues to Aspartic acid. In a specific aspect of this embodiment, the Fc N-glycosylation from said protein of interest is removed using PNGase F.

In some exemplary embodiments, the mass spectrometer is run under native conditions.

### BRIEF DESCRIPTION OF THE DRAWINGS

FIG. 1 illustrates the concepts of a pH gradient and a salt gradient according to an exemplary embodiment.
FIG. 2A shows a pH buffer range of salts used for pH gradients according to an exemplary embodiment.
FIG. 2B shows a pI range of antibodies according to an exemplary embodiment.
FIG. 2C shows pI values of antibodies tested with the AEX-MS method of the invention according to an exemplary embodiment.
FIG. 3 illustrates a high salt-tolerant native AEX-MS system according to an exemplary embodiment.
FIG. 4A shows a trace of a pH gradient in AEX-MS according to an exemplary embodiment.
FIG. 4B shows a trace of a salt gradient in AEX-MS according to an exemplary embodiment.
FIG. 5A shows a native AEX-MS analysis of antibodies of varying pI according to an exemplary embodiment.
FIG. 5B shows a native AEX-MS analysis of antibodies of varying pI according to an exemplary embodiment.
FIG. 5C shows a native AEX-MS analysis of antibodies of varying pI according to an exemplary embodiment.
FIG. 5D shows the pI and effectiveness of AEX separation for each of the antibodies of FIG. 5A-C.
FIG. 6A shows AEX-TICs of antibodies of varying type and pI according to an exemplary embodiment.
FIG. 6B shows a zoomed in view of the AEX-TICs of FIG. 6A according to an exemplary embodiment.
FIG. 7 shows an AEX-TIC of a deglycosylated, FabRICATOR-digested IgG4 mAb according to an exemplary embodiment.
FIG. 8A shows an SCX-TIC of an antibody according to an exemplary embodiment.
FIG. 8B shows an AEX-TIC of an antibody according to an exemplary embodiment.
FIG. 9 illustrates deglycosylation of an antibody using PNGase F according to an exemplary embodiment.
FIG. 10A shows an AEX-TIC comparison of a non-deglycosylated and a deglycosylated antibody according to an exemplary embodiment.
FIG. 10B shows an AEX-TIC comparison of a non-deglycosylated and a deglycosylated antibody according to an exemplary embodiment.
FIG. 10C shows an AEX-TIC comparison of a non-deglycosylated and a deglycosylated antibody according to an exemplary embodiment.
FIG. 11A shows an AEX-TIC comparison of a non-deglycosylated and a deglycosylated antibody according to an exemplary embodiment.
FIG. 11B shows a zoomed-in view of the AEX-TIC of FIG. 11A according to an exemplary embodiment.
FIG. 12 shows an AEX-TIC comparison of a non-deglycosylated and a deglycosylated antibody according to an exemplary embodiment.
FIG. 13 shows an AEX-TIC comparison of a non-deglycosylated and a deglycosylated antibody according to an exemplary embodiment.
FIG. 14A shows an AEX-TIC comparison of a non-deglycosylated method control of an antibody and a non-deglycosylated near acidic fraction of the antibody according to an exemplary embodiment.
FIG. 14B shows a zoomed-in view of the AEX-TICs of FIG. 14A according to an exemplary embodiment.
FIG. 14C shows an AEX-TIC comparison of a deglycosylated method control of an antibody and a deglycosylated near acidic fraction of the antibody according to an exemplary embodiment.
FIG. 14D shows a zoomed-in view of the AEX-TICs of FIG. 14C according to an exemplary embodiment.
FIG. 15A shows an AEX-TIC comparison of a non-deglycosylated antibody from drug substance and a non-deglycosylated total acidic fraction of the antibody according to an exemplary embodiment.
FIG. 15B shows an AEX-TIC comparison of a deglycosylated antibody from drug substance and a deglycosylated total acidic fraction of the antibody according to an exemplary embodiment.
FIG. 16 illustrates Fc charge variants readily separable by AEX-MS, including glycosylation, deamidation and C-terminal lysines, according to an exemplary embodiment.
FIG. 17A shows AEX-TICs of deglycosylated Fc regions of various antibodies according to an exemplary embodiment.
FIG. 17B shows a zoomed-in view of the AEX-TICs of FIG. 17A according to an exemplary embodiment.
FIG. 18A shows an AEX-TIC of a control and a stressed deglycosylated Fc sample according to an exemplary embodiment.
FIG. 18B shows a quantification of the charge variants of FIG. 18A according to an exemplary embodiment.
FIG. 19 illustrates Fc exchange of a bispecific antibody according to an exemplary embodiment.
FIG. 20 shows an AEX-TIC of a non-deglycosylated bsAb FabRICATOR digest according to an exemplary embodiment.

### DETAILED DESCRIPTION

Thorough characterization of biotherapeutic proteins is an essential requirement at all stages of development and final product quality control. Each protein will have several different variant forms due to multiple post-translational modifications that can alter the charge distribution on the surface of the protein. This can be due to a modification which directly alters the charge state or by indirectly altering the accessible surface charge through a conformational change. These modifications can be chromatographically separated using ion-exchange (IEX) analysis.

Methods employing ion exchange chromatography coupled on-line to mass spectrometry have previously been developed in order to characterize charge variants in intact proteins. However, the methods published so far illustrate characterization of proteins with respect to their charge variants, wherein the protein has a pI of less than 6.0. For example, Leblanc *et al.* characterized charge variants of human serum albumin (HSA) (pI = 4.7) using anion exchange chromatography (AEX) coupled online with a MS. See Leblanc et al. J. Chroma. B, 2018, 1095: 87-93.

Fussl *et al.* also reported a pH gradient-based AEX method, which was directly interfaced to a mass spectrometry for characterization of charge variants at the intact protein level under native conditions. Fussl et al. J. Proteome Res., 2019, 18: 3689-3702. However, pH gradient elution on the developed method only facilitated chromatographic selectivity and generic applicability for anionic proteins with pI values <6.5, such as transferrin (pI = 5.2-5.6), ovalbumin (pI = 4.5), and Asialo α-1-AGP (pI = 2.8-3.8). *Id.* at 3690.

Very recently, Van Schaik *et al.* applied AEX-MS to monitoring multiple quality attributes of biopharmaceutical erythropoietin (EPO, pI = 4.4-5.1). All the aforementioned methods employ a pH-gradient elution to separate the proteins and their charge variants.

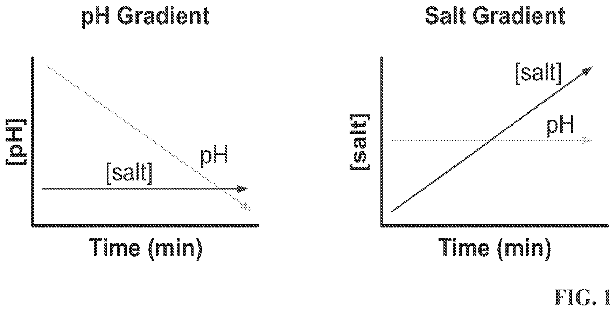

The present invention discloses an AEX method suitable for characterizing charge variants of proteins, which can have pI values of about 6.0 to about 7.0. Two major mechanisms for elution of proteins from AEX column were analyzed to study the separation of charge variants from proteins with a higher pI value than what was previously been published. See, for example FIG. 1. In salt gradient elution, proteins are pushed down the column from exchange site to exchange site by competing with the salt ions in the salt gradient. A focusing effect occurs as the salt concentration increases. With pH gradient elution, proteins will elute from the column when the pH reaches the point where they have little to no charge. A further decrease in pH during the gradient will make the protein more cationic and therefore repelled from the column resin. This is an important difference from salt gradients, as they will then have very little interaction with the rest of the column.

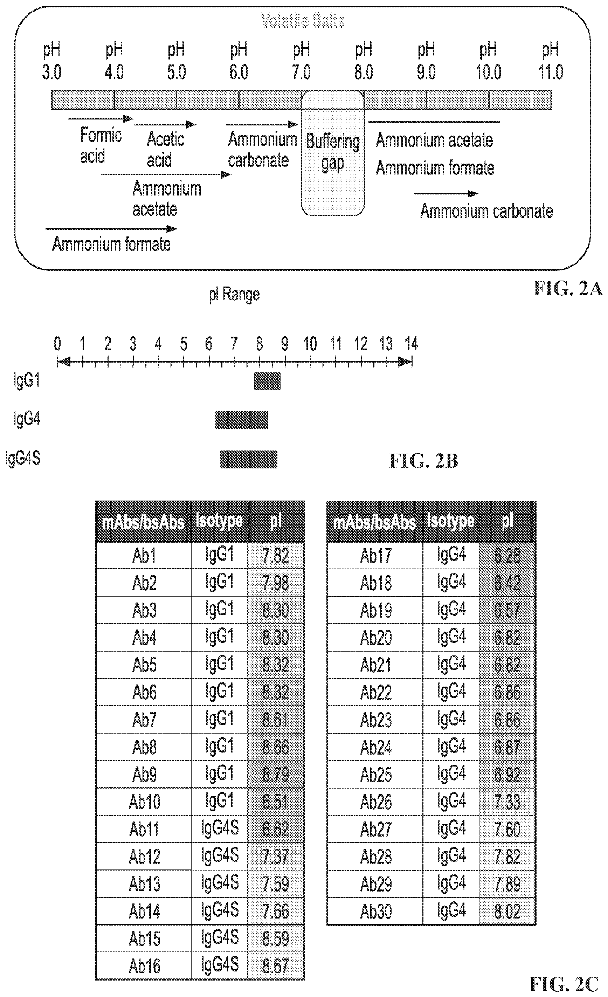

Advantages of pH gradients are also observed with an increase in the sample loading capacity, as the proteins refocus well at the pH of elution, irrespective of the position where they were originally bound on the column. This also compensates the effect of high salt in the sample, thereby reducing sample matrix effects. This routinely used and favored elution method of pH gradient elution was not useful for proteins having pI of more than about 7 as it is difficult to achieve the required pH using buffers which can be compatible with MS devices (i.e., linear pH gradients are difficult to achieve in the desired range of pH over 7.0 using MS-compatible salts, which have a buffering gap near the pI range of antibodies). For example, proteins having higher pI compared to what has been previously published would fall into a buffering gap wherein no MS-compatible salts can be used. See for example, FIG. 2A. Theoretically, this gap makes it difficult for AEX to separate antibodies, due to their relatively high pI, as shown in FIG. 2B. IgG1 antibodies tested tended to have a higher pI relative to IgG4 antibodies, as shown in FIG. 2B and 2C.

This disclosure sets forth a novel native AEX-MS method for characterizing charge variants for proteins by AEX-MS using a salt gradient.

Unless described otherwise, all technical and scientific terms used herein have the same meaning as commonly understood by one of ordinary skill in the art to which this invention belongs. Although any methods and materials similar or equivalent to those described herein can be used in the practice or testing, particular methods and materials are now described. All publications mentioned are hereby incorporated by reference.

The term "a" should be understood to mean "at least one"; and the terms "about" and "approximately" should be understood to permit standard variation as would be understood by those of ordinary skill in the art; and where ranges are provided, endpoints are included.

As used herein, the term "protein" includes any amino acid polymer having covalently linked amide bonds. Proteins comprise one or more amino acid polymer chains, generally known in the art as "polypeptides". "Polypeptide" refers to a polymer composed of amino acid residues, related naturally occurring structural variants, and synthetic non-naturally occurring analogs thereof linked via peptide bonds, related naturally occurring structural variants, and synthetic non-naturally occurring analogs thereof. "Synthetic peptides or polypeptides' refers to a non-naturally occurring peptide or polypeptide. Synthetic peptides or polypeptides can be synthesized, for example, using an automated polypeptide synthesizer. Various solid phase peptide synthesis methods are known. A protein may contain one or multiple polypeptides to form a single functioning biomolecule. A protein can include any of bio-therapeutic proteins, recombinant proteins used in research or therapy, trap proteins and other chimeric receptor Fc-fusion proteins, chimeric proteins, antibodies, monoclonal antibodies, polyclonal antibodies, human antibodies, and bispecific antibodies. In another exemplary aspect, a protein can include antibody fragments, nanobodies, recombinant antibody chimeras, cytokines, chemokines, peptide hormones, and the like. Proteins may be produced using recombinant cell-based production systems, such as the insect bacculovirus system, yeast systems (*e.g.,* Pichia sp.), mammalian systems (*e.g.,* CHO cells and CHO derivatives like CHO-K1 cells). For a review discussing biotherapeutic proteins and their production, see Ghaderi et al., "Production platforms for biotherapeutic glycoproteins. Occurrence, impact, and challenges of non-human sialylation," (BIOTECHNOL. GENET. ENG. REV. 147-175 (2012)). In some exemplary embodiments, proteins comprise modifications, adducts, and other covalently linked moieties. Those modifications, adducts and moieties include for example avidin, streptavidin, biotin, glycans (e.g., N-acetylgalactosamine, galactose, neuraminic acid, N-acetylglucosamine, fucose, mannose, and other monosaccharides), PEG, polyhistidine, FLAGtag, maltose binding protein (MBP), chitin binding protein (CBP), glutathione-S-transferase (GST) myc-epitope, fluorescent labels and other dyes, and the like. Proteins can be classified on the basis of compositions and solubility and can thus include simple proteins, such as, globular proteins and fibrous proteins; conjugated proteins, such as nucleoproteins, glycoproteins, mucoproteins, chromoproteins, phosphoproteins, metalloproteins, and lipoproteins; and derived proteins, such as primary derived proteins and secondary derived proteins.

In some exemplary embodiments, the protein of interest can be an antibody, a bispecific antibody, a multispecific antibody, antibody fragment, monoclonal antibody, or an Fc fusion protein.

The term "antibody," as used herein includes immunoglobulin molecules comprising four polypeptide chains, two heavy (H) chains and two light (L) chains inter-connected by disulfide bonds, as well as multimers thereof (e.g., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L1}). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different exemplary embodiments, the FRs of the anti-big-ET-1 antibody (or antigen-binding portion thereof) may be identical to the human germline sequences, or may be naturally or artificially modified. An amino acid consensus sequence may be defined based on a side-by-side analysis of two or more CDRs. The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, *e.g.,* from full antibody molecules using any suitable standard techniques such as proteolytic digestion or recombinant genetic engineering techniques involving the manipulation and expression of DNA encoding antibody variable and optionally constant domains. Such DNA is known and/or is readily available from, for example, commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

As used herein, an "antibody fragment" includes a portion of an intact antibody, such as, for example, the antigen-binding or variable region of an antibody. Examples of antibody fragments include, but are not limited to, a Fab fragment, a Fab' fragment, a F(ab')2 fragment, a Fc fragment, a scFv fragment, a Fv fragment, a dsFv diabody, a dAb fragment, a Fd' fragment, a Fd fragment, and an isolated complementarity determining region (CDR) region, as well as triabodies, tetrabodies, linear antibodies, single-chain antibody molecules, and multi specific antibodies formed from antibody fragments. Fv fragments are the combination of the variable regions of the immunoglobulin heavy and light chains, and ScFv proteins are recombinant single chain polypeptide molecules in which immunoglobulin light and heavy chain variable regions are connected by a peptide linker. An antibody fragment may be produced by various means. For example, an antibody fragment may be enzymatically or chemically produced by fragmentation of an intact antibody and/or it may be recombinantly produced from a gene encoding the partial antibody sequence. Alternatively or additionally, an antibody fragment may be wholly or partially synthetically produced. An antibody fragment may optionally comprise a single chain antibody fragment. Alternatively or additionally, an antibody fragment may comprise multiple chains that are linked together, for example, by disulfide linkages. An antibody fragment may optionally comprise a multi-molecular complex.

The term "monoclonal antibody" as used herein is not limited to antibodies produced through hybridoma technology. A monoclonal antibody can be derived from a single clone, including any eukaryotic, prokaryotic, or phage clone, by any means available or known in the art. Monoclonal antibodies useful with the present disclosure can be prepared using a wide variety of techniques known in the art including the use of hybridoma, recombinant, and phage display technologies, or a combination thereof.

The term "Fc fusion proteins" as used herein includes part or all of two or more proteins, one of which is an Fc portion of an immunoglobulin molecule, that are not fused in their natural state. Preparation of fusion proteins comprising certain heterologous polypeptides fused to various portions of antibody-derived polypeptides (including the Fc domain) has been described, e.g., by Ashkenazi et al., Proc. Natl. Acad. Sci USA 88: 10535, 1991; Byrn et al., Nature 344:677, 1990; and Hollenbaugh *et al.,* "Construction of Immunoglobulin Fusion Proteins", in Current Protocols in Immunology, Suppl. 4, pages 10.19.1-10.19.11, 1992. "Receptor Fc fusion proteins" comprise one or more of one or more extracellular domain(s) of a receptor coupled to an Fc moiety, which in some embodiments comprises a hinge region followed by a CH2 and CH3 domain of an immunoglobulin. In some embodiments, the Fc-fusion protein contains two or more distinct receptor chains that bind to a single or more than one ligand(s). For example, an Fc-fusion protein is a trap, such as for example an IL-1 trap (e.g., Rilonacept, which contains the IL-1 RAcP ligand binding region fused to the IL-1R1 extracellular region fused to Fc of hIgG1; see U.S. Pat. No. 6,927,004, which is herein incorporated by reference in its entirety), or a VEGF Trap (*e.g.,* Aflibercept, which contains the Ig domain 2 of the VEGF receptor Flt1 fused to the Ig domain 3 of the VEGF receptor Flk1 fused to Fc of hIgGl; *e.g.,* SEQ ID NO:1; see U.S. Pat. Nos. 7,087,411 and 7,279,159, which are herein incorporated by reference in their entirety)

As used herein, the general term "post-translational modifications" or "PTMs" refer to covalent modifications that polypeptides undergo, either during (co-translational modification) or after (post-translational modification) their ribosomal synthesis. PTMs are generally introduced by specific enzymes or enzyme pathways. Many occur at the site of a specific characteristic protein sequence (signature sequence) within the protein backbone. Several hundred PTMs have been recorded, and these modifications invariably influence some aspect of a protein's structure or function (Walsh, G. "Proteins" (2014) second edition, published by Wiley and Sons, Ltd., ISBN: 9780470669853). The various post-translational modifications include, but are not limited to, cleavage, N-terminal extensions, protein degradation, acylation of the N-terminus, biotinylation (acylation of lysine residues with a biotin), amidation of the C-terminal, glycosylation, iodination, covalent attachment of prosthetic groups, acetylation (the addition of an acetyl group, usually at the N-terminus of the protein), alkylation (the addition of an alkyl group (e.g. methyl, ethyl, propyl) usually at lysine or arginine residues), methylation, adenylation, ADP-ribosylation, covalent cross links within, or between, polypeptide chains, sulfonation, prenylation, Vitamin C dependent modifications (proline and lysine hydroxylations and carboxy terminal amidation), Vitamin K dependent modification wherein Vitamin K is a cofactor in the carboxylation of glutamic acid residues resulting in the formation of a γ-carboxyglutamate (a glu residue), glutamylation (covalent linkage of glutamic acid residues), glycylation (covalent linkage glycine residues), glycosylation (addition of a glycosyl group to either asparagine, hydroxylysine, serine, or threonine, resulting in a glycoprotein), isoprenylation (addition of an isoprenoid group such as farnesol and geranylgeraniol), lipoylation (attachment of a lipoate functionality), phosphopantetheinylation (addition of a 4'-phosphopantetheinyl moiety from coenzyme A, as in fatty acid, polyketide, non-ribosomal peptide and leucine biosynthesis), phosphorylation (addition of a phosphate group, usually to serine, tyrosine, threonine or histidine), and sulfation (addition of a sulfate group, usually to a tyrosine residue). The post-translational modifications that change the chemical nature of amino acids include, but are not limited to, citrullination (the conversion of arginine to citrulline by deimination), and deamidation (the conversion of glutamine to glutamic acid or asparagine to aspartic acid). The post-translational modifications that involve structural changes include, but are not limited to, formation of disulfide bridges (covalent linkage of two cysteine amino acids) and proteolytic cleavage (cleavage of a protein at a peptide bond). Certain post-translational modifications involve the addition of other proteins or peptides, such as ISGylation (covalent linkage to the ISG15 protein (Interferon-Stimulated Gene)), SUMOylation (covalent linkage to the SUMO protein (Small Ubiquitin-related MOdifier)) and ubiquitination (covalent linkage to the protein ubiquitin). See European Bioinformatics Institute Protein Information ResourceSIB Swiss Institute of Bioinformatics, EUROPEAN BIOINFORMATICS INSTITUTE DRS - DROSOMYCIN PRECURSOR - DROSOPHILA MELANOGASTER (FRUIT FLY) - DRS GENE & PROTEIN, http://www.uniprot.org/docs/ptmlist (last visited Jan 15, 2019) for a more detailed controlled vocabulary of PTMs curated by UniProt.

As used herein, the term "charge variants", refers to the full complement of product variants including, but not limited to acidic species, and basic species (e.g., Lys variants). In certain embodiments, such variants can include product aggregates and/or product fragments, to the extent that such aggregation and/or fragmentation results in a product charge variation.

As used herein, the term "acidic species" refers to the variants of a protein which are characterized by an overall acidic charge. Exemplary acidic species can include, but are not limited to, deamidation variants, afucosylation variants, oxidation variants, methylglyoxal (MGO) variants, glycation variants, and citric acid variants. Exemplary structure variants include, but are not limited to, glycosylation variants and acetonation variants. Exemplary fragmentation variants include any modified protein species from the target molecule due to dissociation of peptide chain, enzymatic and/or chemical modifications, including, but not limited to, Fc and Fab fragments, fragments missing a Fab, fragments missing a heavy chain variable domain, C-terminal truncation variants, variants with excision of N-terminal Asp in the light chain, and variants having N-terminal truncation of the light chain. Other acidic species variants include variants comprising unpaired disulfides, host cell proteins, and host nucleic acids, chromatographic materials, and media components. Commonly, acidic species elute later than the main peak during CEX or later than the main peak during AEX analysis

As used herein, the term "basic species" refers to the variants of a protein, for example, an antibody or antigen-binding portion thereof, which are characterized by an overall basic charge, relative to the primary charge variant species present within the protein. Exemplary basic species can include, but are not limited to, lysine variants, isomerization of aspartic acid, succinimide formation at asparagine, methionine oxidation, amidation, incomplete disulfide bond formation, mutation from serine to arginine, aglycosylation, fragmentation and aggregation. Commonly, basic species elute later than the main peak during CEX or earlier than the main peak during AEX analysis.

As used herein, "ion exchange chromatography" can refer to separations including any method by which two substances are separated based on differences in their respective ionic charges, either on the molecule of interest and/or chromatographic material as a whole or locally on specific regions of the molecule of interest and/or chromatographic material, and thus can employ either cationic exchange material or anionic exchange material. Ion exchange chromatography separates molecules based on differences between the local charges of the molecules of interest and the local charges of the chromatographic material. A packed ion-exchange chromatography column or an ion-exchange membrane device can be operated in a bind-elute mode, a flowthrough mode, or a hybrid mode. After washing the column or the membrane device with an equilibration buffer or another buffer, product recovery can be achieved by increasing the ionic strength (*i.e.,* conductivity) of the elution buffer to compete with the solute for the charged sites of the ion exchange matrix. Changing the pH and thereby altering the charge of the solute can be another way to achieve elution of the solute. The change in conductivity or pH may be gradual (gradient elution) or stepwise (step elution). Anionic or cationic substituents may be attached to matrices in order to form anionic or cationic supports for chromatography. Non-limiting examples of anionic exchange substituents include diethylaminoethyl (DEAE), quaternary aminoethyl (QAE) and quaternary amine (Q) groups.

As used herein, the term "mass spectrometer" includes a device capable of identifying specific molecular species and measuring their accurate masses. The term is meant to include any molecular detector into which a polypeptide or peptide may be eluted for detection and/or characterization. A mass spectrometer can include three major parts: the ion source, the mass analyzer, and the detector. The role of the ion source is to create gas phase ions. Analyte atoms, molecules, or clusters can be transferred into gas phase and ionized either concurrently (as in electrospray ionization). The choice of ion source depends heavily on the application.

In some embodiments, the mass spectrometer can be an electrospray-mass spectrometer.

As used herein, the term "electrospray ionization" or "ESI" refers to the process of spray ionization in which either cations or anions in solution are transferred to the gas phase via formation and desolvation at atmospheric pressure of a stream of highly charged droplets that result from applying a potential difference between the tip of the electrospray needle containing the solution and a counter electrode.

In some exemplary embodiments, the electrospray ionization mass spectrometer can be a nano-electrospray ionization mass spectrometer.

The term "nanoelectrospray" or "nanospray" as used herein refers to electrospray ionization at a very low solvent flow rate, typically hundreds of nanoliters per minute of sample solution or lower, often without the use of an external solvent delivery. The electrospray infusion setup forming a nanoelectrospray can use a static nanoelectrospray emitter or a dynamic nanoelectrospray emitter. A static nanoelectrospray emitter performs a continuous analysis of small sample (analyte) solution volumes over an extended period of time. A dynamic nanoelectrospray emitter uses a capillary column and a solvent delivery system to perform chromatographic separations on mixtures prior to analysis by the mass spectrometer.

As used herein, the term "mass analyzer" includes a device that can separate species, that is, atoms, molecules, or clusters, according to their mass. Non-limiting examples of mass analyzers that could be employed for fast protein sequencing are time-of-flight (TOF), magnetic / electric sector, quadrupole mass filter (Q), quadrupole ion trap (QIT), orbitrap, Fourier transform ion cyclotron resonance (FTICR), and also the technique of accelerator mass spectrometry (AMS).

In some exemplary embodiments, mass spectrometry can be performed under native conditions.

As used herein, the term "native conditions" or "native MS" or "native ESI- MS" can include a performing mass spectrometry under conditions that preserve no-covalent interactions in an analyte. For detailed review on native MS, refer to the review: Elisabetta Boeri Erba & Carlo Petosa, The emerging role of native mass spectrometry in characterizing the structure and dynamics of macromolecular complexes, 24 PROTEIN SCIENCE1176-1192 (2015). Some of the distinctions between native ESI and regular ESI are illustrated in table 1 and FIG. 1 (Hao Zhang et al., Native mass spectrometry of photosynthetic pigment-protein complexes, 587 FEBS Letters 1012-1020 (2013)).

In some exemplary embodiments, the mass spectrometer can be a tandem mass spectrometer.

As used herein, the term "tandem mass spectrometry" includes a technique where structural information on sample molecules is obtained by using multiple stages of mass selection and mass separation. A prerequisite is that the sample molecules can be transferred into gas phase and ionized intact and that they can be induced to fall apart in some predictable and controllable fashion after the first mass selection step. Multistage MS/MS, or MSⁿ, can be performed by first selecting and isolating a precursor ion (MS²), fragmenting it, isolating a primary fragment ion (MS³), fragmenting it, isolating a secondary fragment (MS⁴), and so on as long as one can obtain meaningful information or the fragment ion signal is detectable. Tandem MS have been successfully performed with a wide variety of analyzer combinations. What analyzers to combine for a certain application is determined by many different factors, such as sensitivity, selectivity, and speed, but also size, cost, and availability. The two major categories of tandem MS methods are tandem-in-space and tandem-in-time, but there are also hybrids where tandem-in-time analyzers are coupled in space or with tandem-in-space analyzers. A tandem-in-space mass spectrometer comprises an ion source, a precursor ion activation device, and at least two non-trapping mass analyzers. Specific m/z separation functions can be designed so that in one section of the instrument ions are selected, dissociated in an intermediate region, and the product ions are then transmitted to another analyzer for m/z separation and data acquisition. In tandem-in-time mass spectrometer ions produced in the ion source can be trapped, isolated, fragmented, and m/z separated in the same physical device.

The peptides identified by the mass spectrometer can be used as surrogate representatives of the intact protein and their post-translational modifications. They can be used for protein characterization by correlating experimental and theoretical MS/MS data, the latter generated from possible peptides in a protein sequence database. The characterization can include, but is not limited, to sequencing amino acids of the protein fragments, determining protein sequencing, determining protein de novo sequencing, locating post-translational modifications, or identifying post translational modifications, or comparability analysis, or combinations thereof.

As used herein, the term "database" refers to bioinformatic tools which provide the possibility of searching the uninterpreted MS-MS spectra against all possible sequences in the database(s). Non-limiting examples of such tools are Mascot (http://www.matrixscience.com), Spectrum Mill (http://www.chem.agilent.com), PLGS (http://www.waters.com), PEAKS (http://www.bioinformaticssolutions.com), Proteinpilot (http://download.appliedbiosystems.com//proteinpilot), Phenyx (http://www.phenyx-ms.com), Sorcerer (http://www.sagenresearch.com), OMSSA (http://www.pubchem.ncbi.nlm.nih.gov/omssa/), X!Tandem (http://www.thegpm.org/T ANDEM/), Protein Prospector (http://www. http://prospector.ucsf.edu/prospector/mshome.htm), Byonic (https://www.proteinmetrics.com/products/byonic) or Sequest (http://fields.scripps.edu/sequest).

As used herein, the term "digestion" refers to hydrolysis of one or more peptide bonds of a protein. There are several approaches to carrying out digestion of a protein in a sample using an appropriate hydrolyzing agent, for example, enzymatic digestion or non-enzymatic digestion.

As used herein, the term "hydrolyzing agent" refers to any one or combination of a large number of different agents that can perform digestion of a protein. Non-limiting examples of hydrolyzing agents that can carry out enzymatic digestion include trypsin, endoproteinase Arg-C, endoproteinase Asp-N, endoproteinase Glu-C, outer membrane protease T (OmpT), immunoglobulin-degrading enzyme of Streptococcus pyogenes (IdeS), chymotrypsin, pepsin, thermolysin, papain, pronase, and protease from *Aspergillus Saitoi.* Non-limiting examples of hydrolyzing agents that can carry out non-enzymatic digestion include the use of high temperature, microwave, ultrasound, high pressure, infrared, solvents (non-limiting examples are ethanol and acetonitrile), immobilized enzyme digestion (IMER), magnetic particle immobilized enzymes, and on-chip immobilized enzymes. For a recent review discussing the available techniques for protein digestion see Switazar et al., "Protein Digestion: An Overview of the Available Techniques and Recent Developments" (J. Proteome Research 2013, 12, 1067-1077). One or a combination of hydrolyzing agents can cleave peptide bonds in a protein or polypeptide, in a sequence-specific manner, generating a predictable collection of shorter peptides.

### EXAMPLES

IgG1- and IgG4-based therapeutic mAb samples were either directly analyzed or treated with PNGase F and/or FabRICATOR before being injected and separated on a YMC BioPro QA-F SAX column (4.6 x 100 mm). A previously reported native LC-MS platform was applied to accommodate the analytical flowrate (0.4 mL/min) with nanoelectrospray ionization (NSI). Ammonium acetate-based mobile phases ranging from 10 to 300 mM were used to develop salt-based gradient for mAb elution. To elucidate variant peaks resulting from either site-specific deamidation or deglycosylation reactions, offline fractionation and tryptic peptide mapping analyses were performed. Synthetic peptides were also used to differentiate deamidation products (e.g., iso-Asp vs Asp) based on retention time alignment.

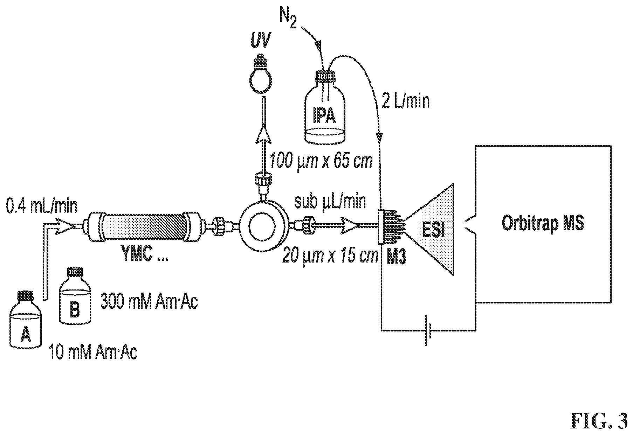

To develop the method of the present invention, AEX was first investigated using either pH or salt gradient modes, as illustrated in FIG. 1. The system used was a Dionex Ultimate 3000 HPLC - Q Exactive UHMR. An exemplary system and method for AEX-MS is described in Yan et al., 2020, J Am Soc Mass Spectrom, 31:2171-2179, which is hereby incorporated by reference, and illustrated in FIG. 3.

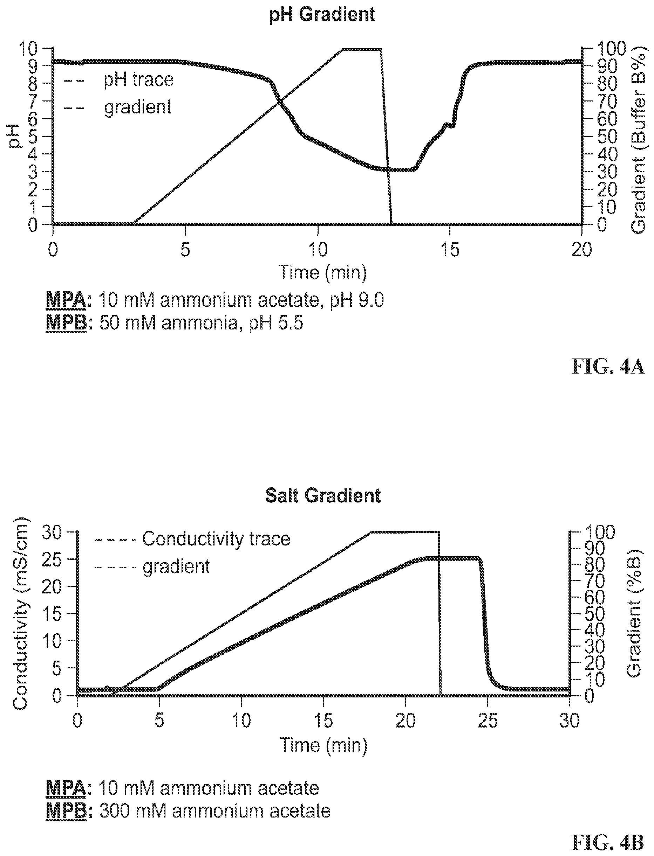

Experiments using AEX-MS with a pH gradient or a salt gradient are shown in FIG. 4A and 4B. A linear pH gradient with the desired pH range cannot be achieved for AEX analysis of mAbs using MS-compatible ammonia-based mobile phases. However, a linear salt gradient can be achieved using MS-compatible mobile phases. The developed native LC-MS platform can tolerate salt concentrations up to 600 mM.

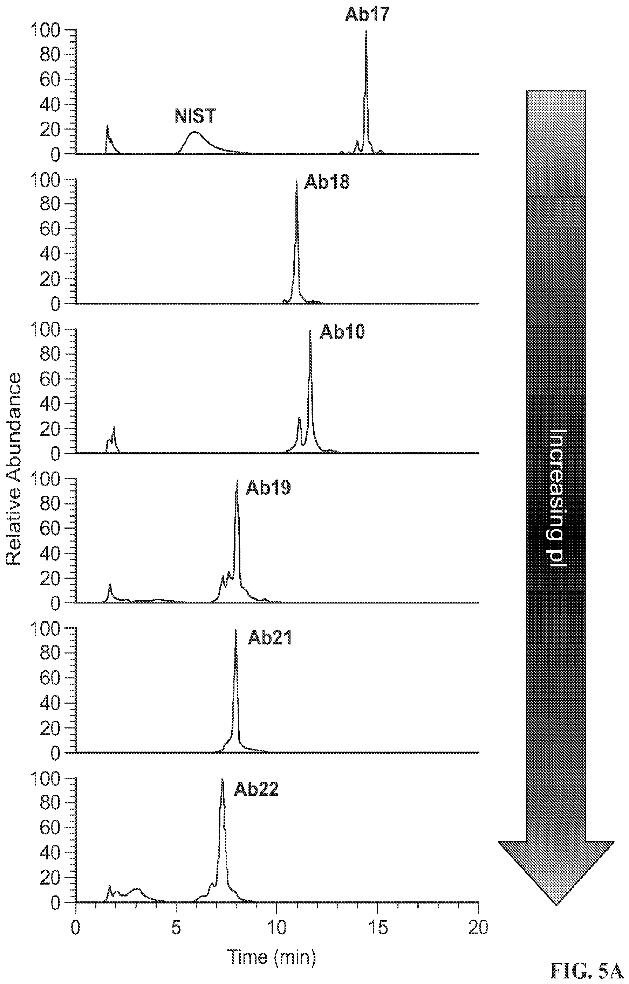

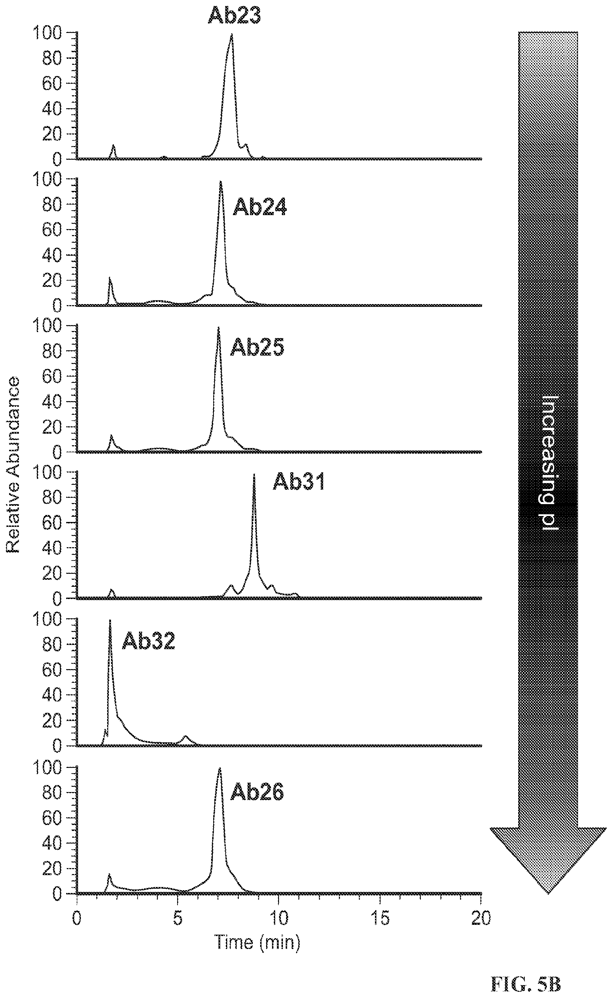

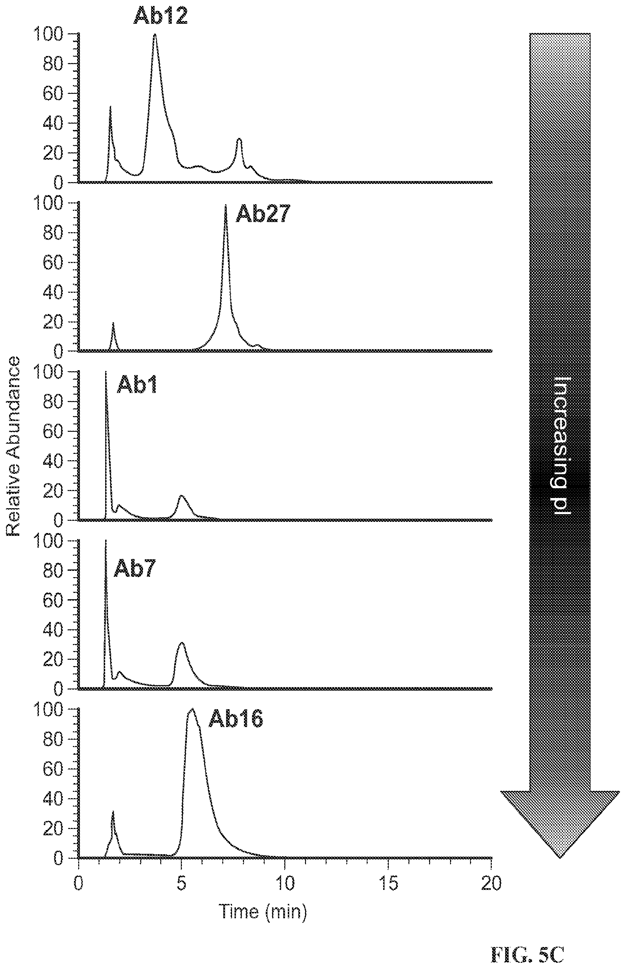

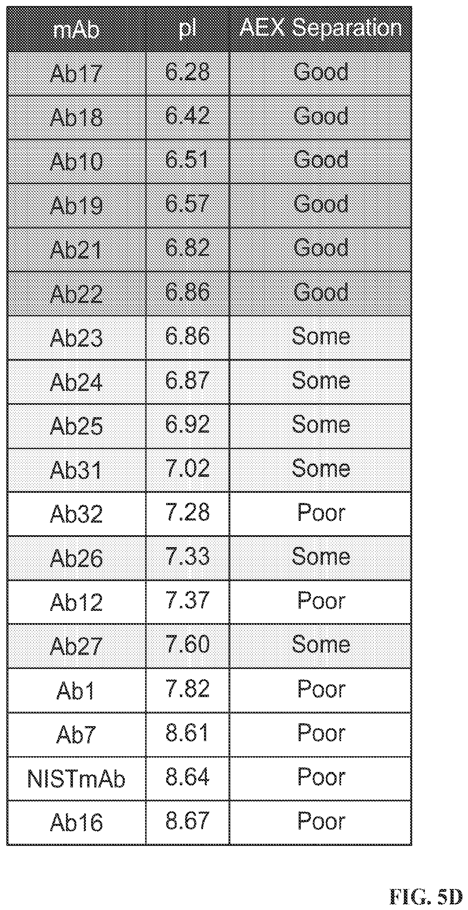

A wide range of antibodies were tested using the AEX-MS system to determine which mAbs would most benefit from AEX separation. AEX TICs of tested antibodies are shown in order of increasing pI in FIG. 5A, 5B, and 5C, with a summary of the results in 5D. Generally, mAbs with relatively lower pI's (pH < 7) had better AEX separation.

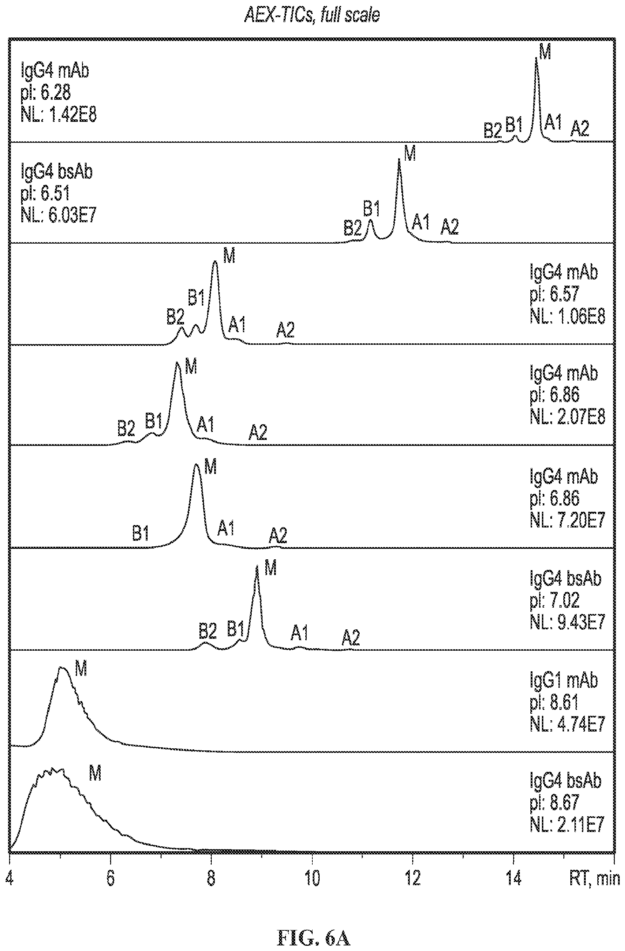

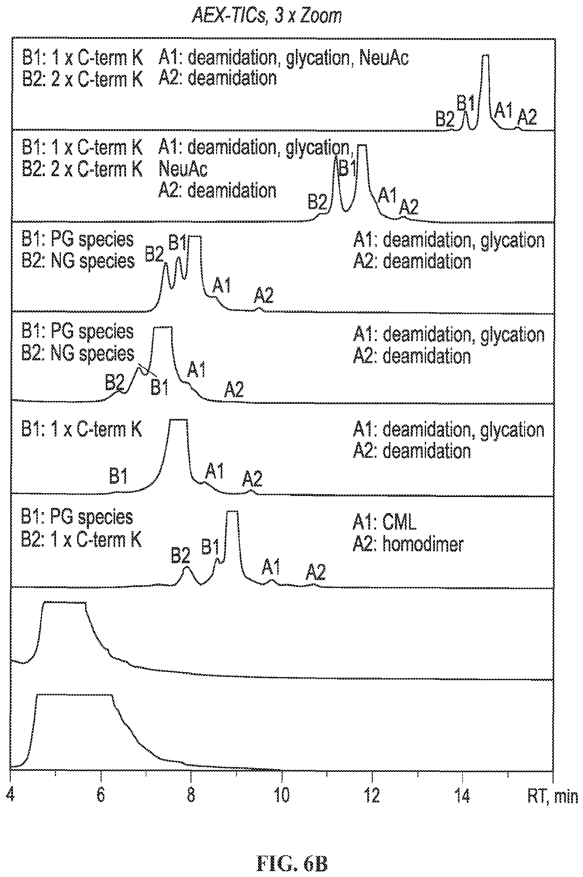

Another comparison of mAbs and bsAbs was conducted using the AEX-MS method of the invention. Full-scale AEX-TICs are shown in FIG. 6A and 3x zoom AEX-TICs are shown in FIG. 6B. The native AEX-MS method was shown to be suitable for most IgG4 molecules with moderate pI, allowing for the identification of various acidic and basic species, and less useful for IgG1 mAbs with high pI. Generally, AEX separation improved for molecules with lower pI. Common acidic variants observed included deamidation, glycation, and NeuAc. Common basic variants observed included partial and non-glycosylated species, and species with C-terminal lysines.

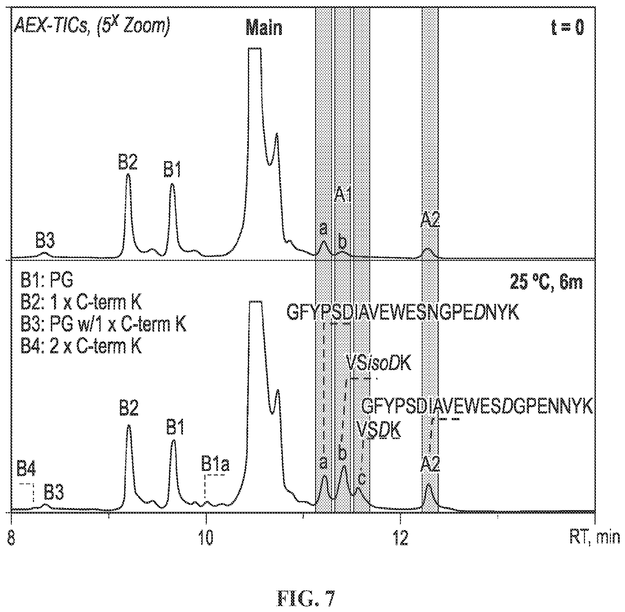

Further analysis was conducted on deglycosylated IgG4 mAbs that were treated with IdeS (FabRICATOR^{®} from Genovis) to perform Fc attribute monitoring, as shown in FIG. 7. Specific acidic peaks were found to correlate to site-specific deamidation. Identities were confirmed by fractionating a deglycosylated and FabRICATOR^{®}-digested thermally-stressed IgG4 sample, performing subsequent reduced peptide mapping, and comparing the results against synthetic peptides.

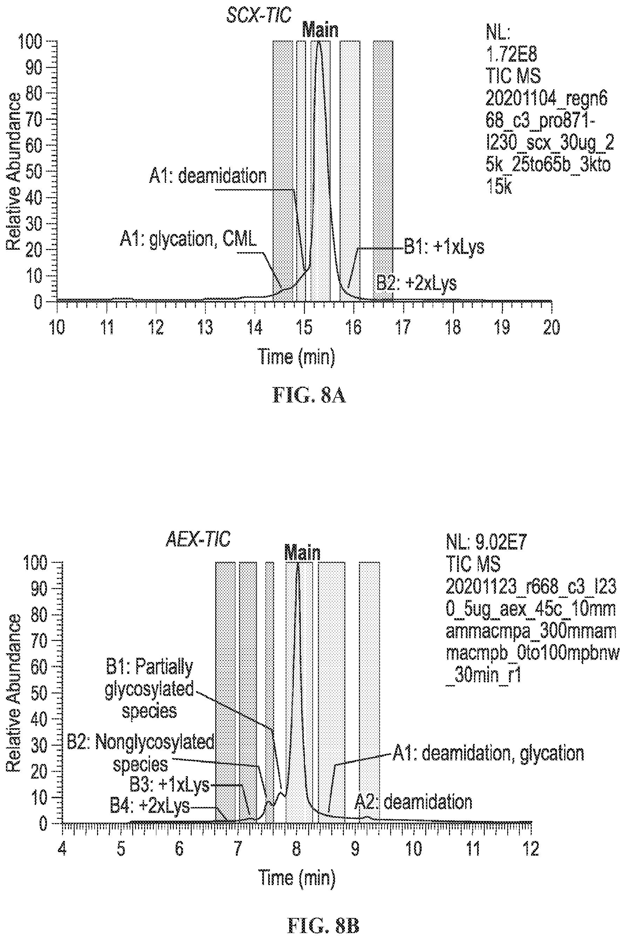

A comparison was made between SCX-MS and AEX-MS for Ab21, as shown in FIG. 8A versus 8B. AEX-MS was able to identify a greater number of variants compared to SCX-MS, demonstrating that for proteins with lower pI's, AEX can be a useful alternative to SCX.

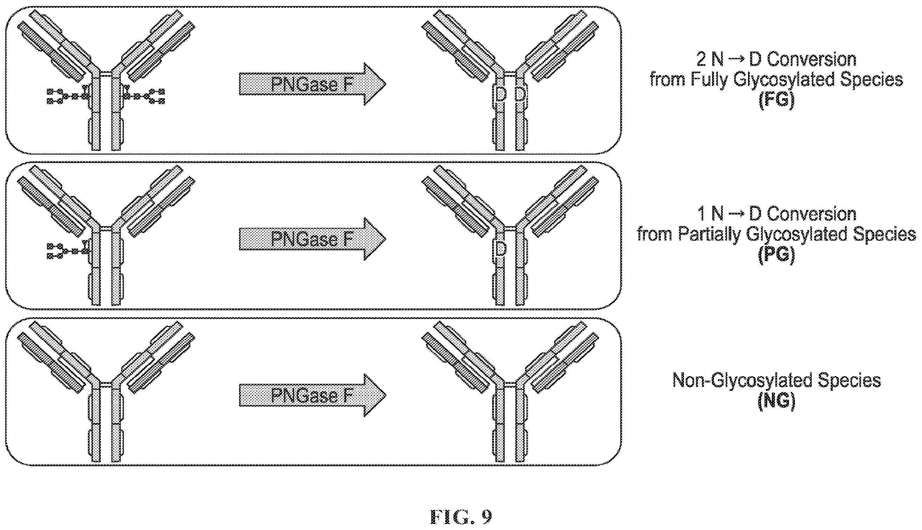

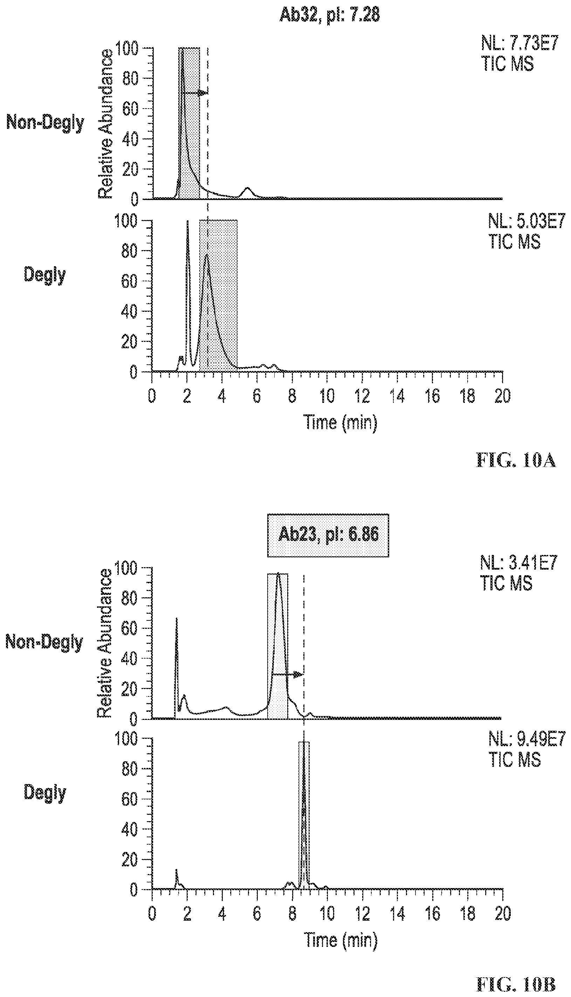

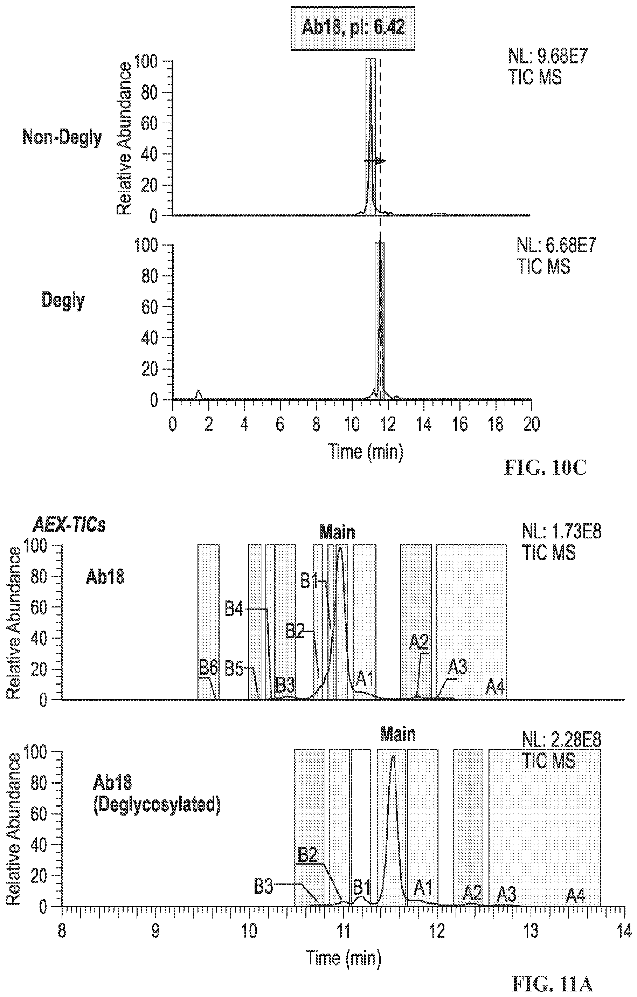

Further improvements were made to the method of the present invention. AEX separation was improved by deglycosylating the analyte antibody with PNGase F, lowering the pI of the sample, as illustrated in FIG. 9. Non-deglycosylated and deglycosylated antibodies were subjected to AEX-MS analysis and the AEX-TICs were compared. Deglycosylation lowered mAb pI, resulting in later elution and improved AEX separation and resolution, as shown in FIG. 10A-C. For mAbs with relatively high pI's (pH > 7), the deglycosylation step can help the retention of the molecule, but the improvement in resolution was not as significant as for mAbs with lower pI's.

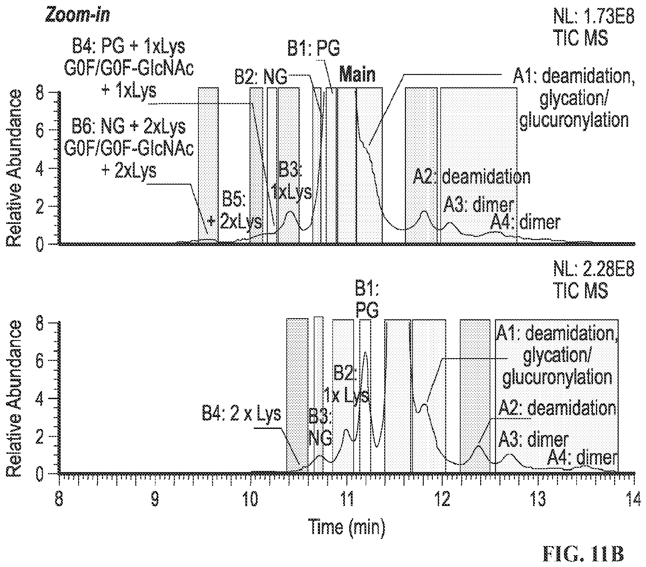

A further comparison of non-deglycosylated and deglycosylated mAb subjected to AEX-MS is shown in FIG. 11A, with a zoom in in FIG. 11B. The method was able to detect a large number of basic and acidic species and differentiate different protein modifications. After deglycosylation, overall resolution was improved. The basic variants detected included C-terminal lysines and converted partially glycosylated species. The acidic variants detected included deamidation, glycation/glucuronylation, and high molecular weight (HMW) species.

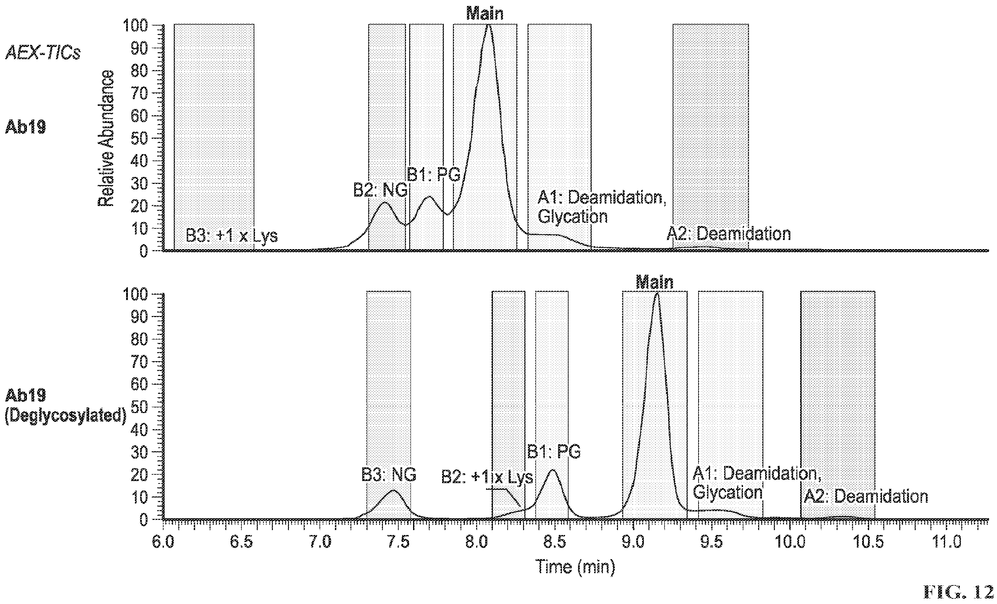

A similar comparison was carried out for another antibody as shown in FIG. 12. Again, AEX resolution was greatly improved after deglycosylation. Basic variants detected included C-terminal lysines, non-glycosylated species, and converted partially glycosylated species. Acidic variants detected included deamidation and glycation/glucuronylation species.

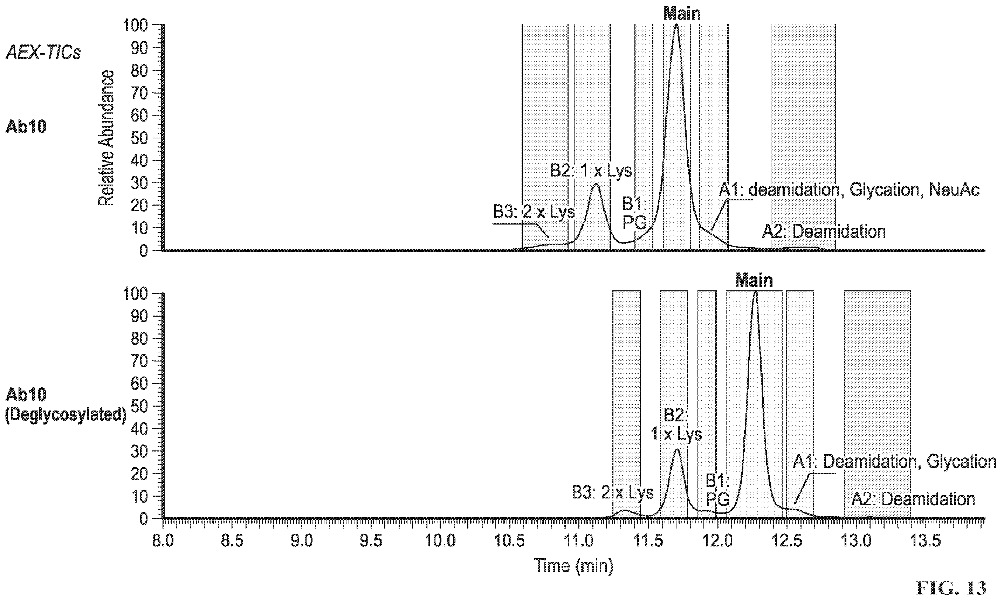

AEX-MS analysis with and without deglycosylation was carried out for another antibody as shown in FIG. 13. Again, AEX resolution was greatly improved after deglycosylation. Basic variants detected included C-terminal lysine and converted partially glycosylated species. Acidic variants detected included deamidation, glycation/glucuronylation, and NeuAc species.

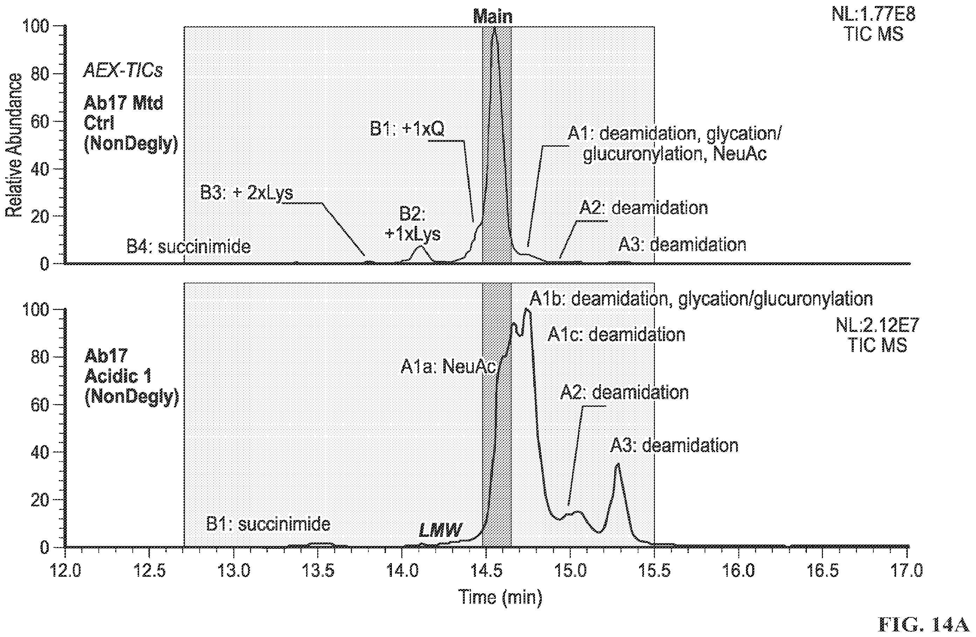

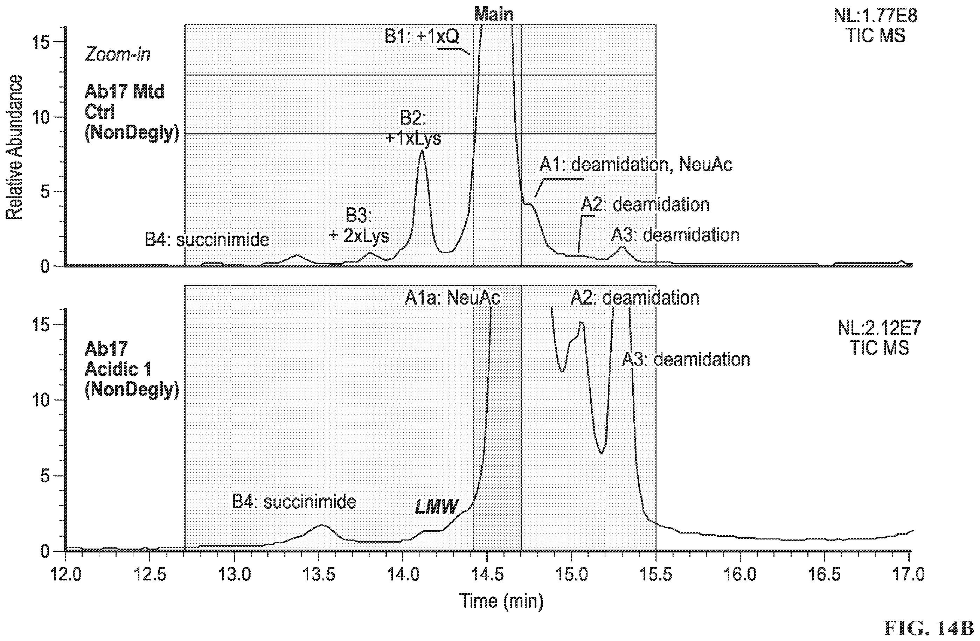

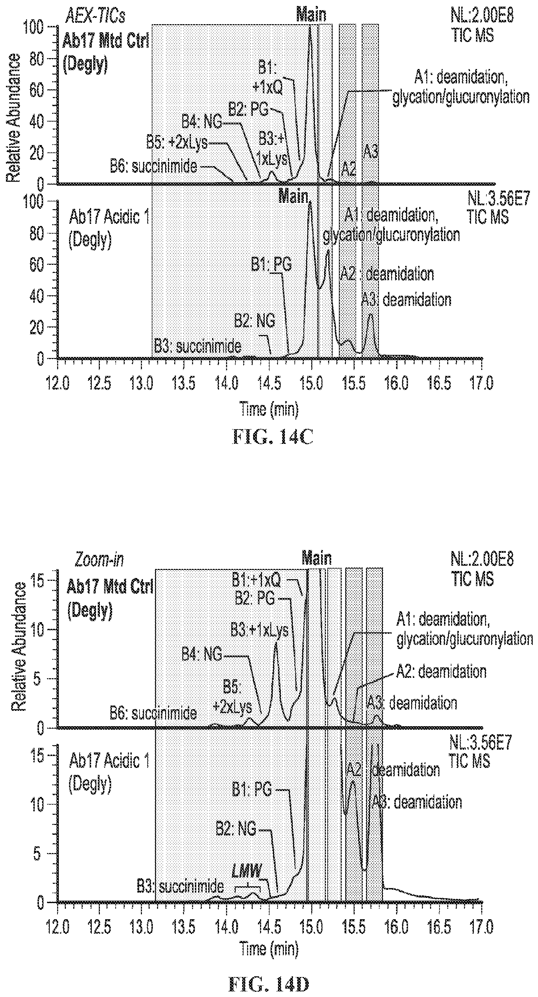

There are a variety of potential applications for the method of the invention. For example, AEX-MS was used for in-depth characterization of enriched charge variants as shown in FIG. 14. Method control samples (Mtd ctrl) were compared to fractionated near acidic species (Acidic 1) in non-deglycosylated and glycosylated samples and subjected to AEX-MS analysis. Basic variants observed in the method control include succinimide and C-terminal lysine, as shown in FIG. 14A and 14B. Acidic variants observed include deamidation, glycation/glucuronylation and sialic acid (NeuAc). The method was capable of further separating charge variants into site-specific deamidation sites. After deglycosylation, additional succinimide peaks were also identified, as shown in FIG. 14C and 14D.

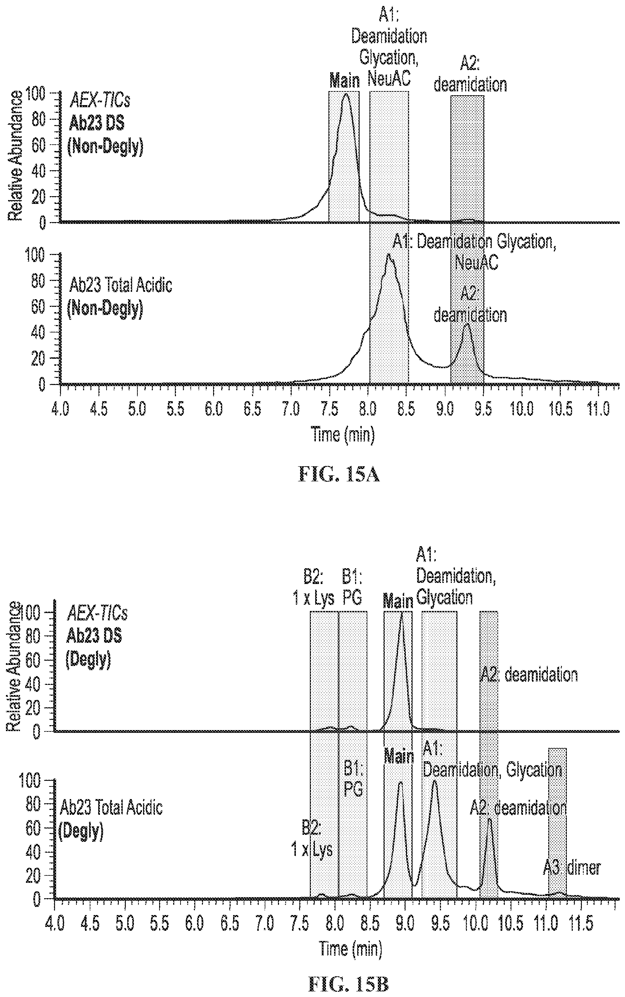

A different enriched charged variant analysis was carried out on another antibody as shown in FIG. 15. Antibody drug substance was compared to a total acidic species fraction. The total acidic pool consists of acidic variants such as deamidation, glycation/glucuronylation and sialic acid (NeuAc), as shown in FIG. 15A. The acidic variants could be further separated into site-specific deamidation sites. After deglycosylation, additional resolution of HMW species was observed, as shown in FIG. 15B

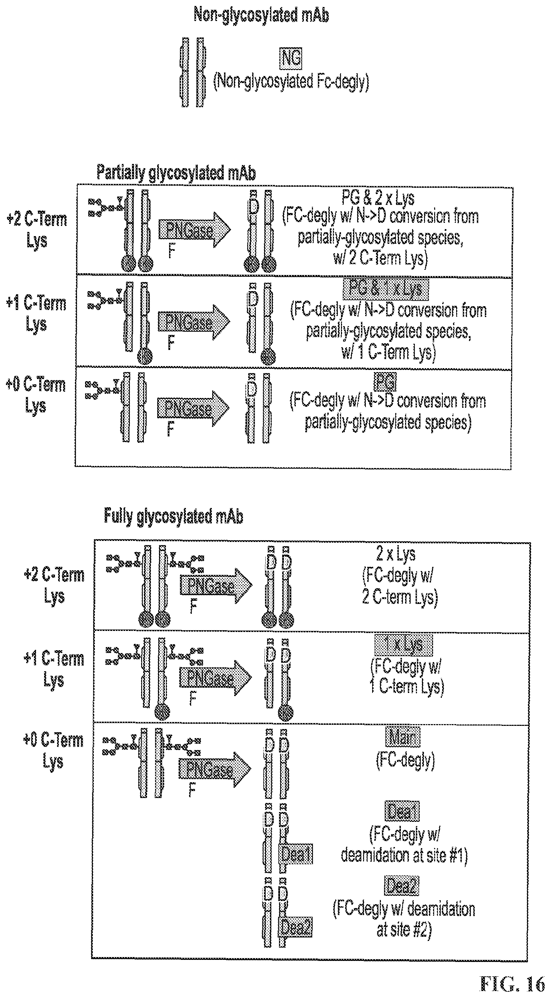

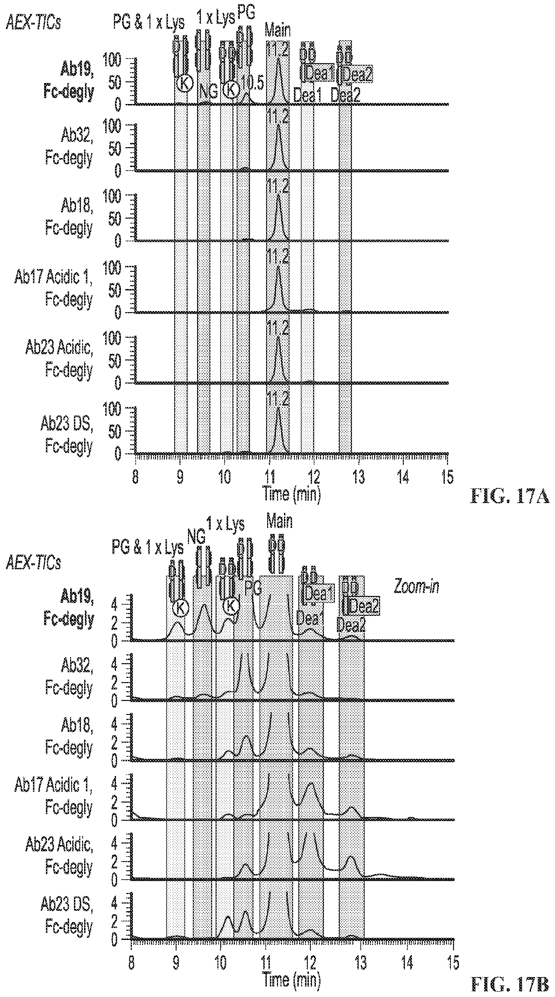

A second application explored was the use of AEX-MS for high-resolution Fc attribute monitoring. After deglycosylation, AEX-MS can simultaneously monitor glycosylation states, C-terminal lysines, and site-specific deamidation, as illustrated in FIG. 16. Because the Fc region of related antibodies, for example IgG4s, is shared, analysis of Fc attributes provides representative information on a wide range of antibodies. An exemplary analysis of a range of proteins is shown in FIG. 17A and 17B, with different peaks representing variants in glycosylation, C-terminal lysines and site-specific deamidation. The method was demonstrated to be broadly applicable to IgG4 antibodies.

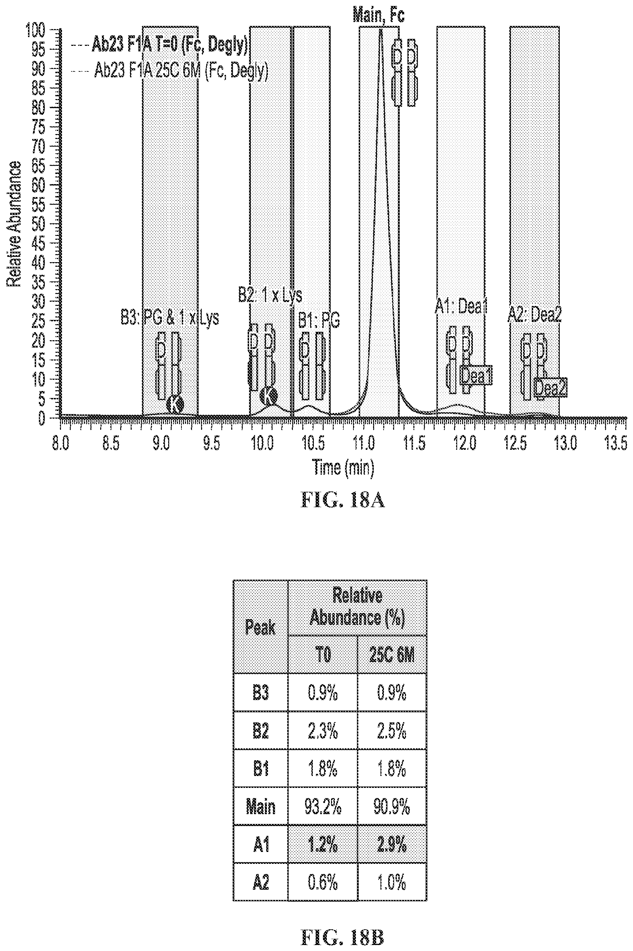

This analysis was further applied to monitor stability samples, as shown in FIG. 18. An AEX-TIC showing a separation of modified antibody species is shown in FIG. 18A, with two overlapping traces indicating a naive sample (in black, lower trace) and a stressed sample (in red, higher trace). The stressed sample was stored at 25° C for 6 months prior to analysis. The abundance of different charged species can be compared and quantified as shown in FIG. 18B. In this example, use of the AEX-MS method of the present invention allowed for the discovery that acidic variants of the antibody (see, for example, A1) increased after thermal stress. The site-specific deamidation site A1 likely correlates with VSNK abased on previous studies.

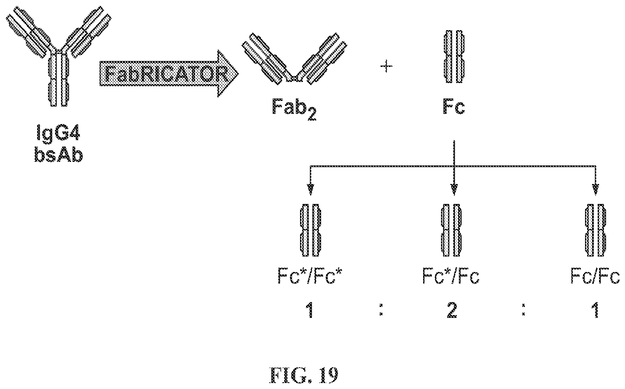

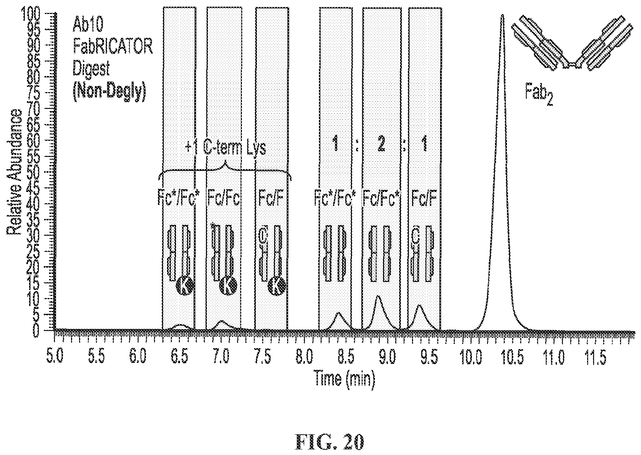

A further application explored is using AEX-MS to monitor Fc and Fc* exchange in a bispecific antibody. It is known that IgG4 can undergo rapid Fc exchange after removing the hinge disulfide bonds. For a bsAb, the Fc and Fc* will exchange into a 1:2:1 ratio of Fc*/Fc* : Fc*/Fc : Fc/Fc products after FabRICATOR^{®} digestion, as illustrated in FIG. 19. The AEX-MS method of the present invention was applied to a bsAb FaBRICATOR^{®} digest and proved sensitive enough to detect Fc exchange and separate Fc species in the predicted 1:2:1 ratio as shown in FIG. 20. In this example Fc* exhibited much higher C-terminal lysine compared to Fc.

A salt-gradient based native AEX-MS method has been developed that can be potentially applied to a range of protein analysis, including charge variant analysis of mAbs with relatively low pI's. The AEX separation profile and resolution of mAbs can be further improved by deglycosylation. AEX is very sensitive to glycosylation states, as well as C-terminal lysine and deamidation. Common basic variants observed in AEX-MS include C-terminal lysine, non-glycosylated or partially glycosylated variants, and succinimide. Common acidic variants observed in AEX-MS include NeuAc, deamidation, glycation and glucuronylation.

AEX can be used orthogonally for in-depth characterization of enriched charge variant samples at BLA stage. AEX-MS can also be used for high-resolution Fc attribute monitoring, and in particular for monitoring site-specific deamidations.

## Claims

1. A method for characterizing at least one charge variant of a protein of interest having an isoelectric point (pI) value of about 6.2 to about 7.0, the method comprising:
a. loading a sample comprising a protein of interest and at least one charge variant of the protein of interest to an anion-exchange chromatography (AEX) column coupled on-line to mass spectrometry;
b. applying an increasing salt concentration gradient to the loaded AEX column to obtain an eluate, without applying a pH gradient, wherein the salt gradient is an elution gradient ranging from about 10 mM to about 600 mM of an ammonium salt;
c. collecting at least one fraction from the eluate of step b); and
d. analyzing the at least one fraction by mass spectrometry to characterize the at least one charge variant of the protein of interest.

2. The method of claim 1, further comprising deglycosylating the sample prior to loading the sample to the AEX column.

3. The method of claim 1 or claim 2, wherein the ammonium salt concentration gradient is an elution gradient ranging from about 10 mM to about 300 mM.

4. The method of any one of claims 1-3, wherein the increasing ammonium salt concentration gradient is applied linearly.

5. The method of any one of claims 1-4, further comprising monitoring the ultraviolet absorbance of the eluate from step b) and collecting the at least one fraction that is eluted prior to or after elution of the protein of interest.

6. The method of any one of claims 1-5, wherein the protein of interest is an Immunoglobulin G subclass 4 (IgG4-) based monoclonal antibody.

7. The method of any one of claims 1-6, wherein the protein of interest is a bispecific monoclonal antibody.

8. The method of any one of claims 1-7, wherein the at least one charge variant is a deamidation, glycation, glucuronylation, high molecular weight species, C-terminal lysine, or glycosylated species of the protein of interest.

9. The method of any one of claims 1-8, further comprising removing an Fc N-glycosylation from the protein of interest prior to loading the sample on the AEX column.

10. The method of any one of claims 1-9, further comprising digesting the sample prior to loading the sample on the AEX column.

11. The method of claim 10, wherein the sample is digested with an Immunoglobulin G-degrading enzyme of Streptococcus pyogenes (IdeS) or a variant thereof .

12. The method of any one of claims 1-11, wherein the mass spectrometer is run under native conditions.

## Patentansprüche

1. Verfahren zur Charakterisierung mindestens einer Ladungsvariante eines Proteins von Interesse mit einem Isoelektrischer-Punkt(pI)-Wert von etwa 6,2 bis etwa 7,0, wobei das Verfahren Folgendes umfasst:
a. Laden einer Probe, die ein Protein von Interesse und mindestens eine Ladungsvariante des Proteins von Interesse umfasst, auf eine Anionenaustauschchromatographie(AEX)-Säule, die online an Massenspektrometrie gekoppelt ist;
b. Auftragen eines zunehmenden Salzkonzentrationsgradienten auf die beladene AEX-Säule, so dass ein Eluat erhalten wird, ohne einen pH-Gradienten anzuwenden, wobei es sich bei dem Salzgradienten um einen Elutionsgradienten im Bereich von etwa 10 mM bis etwa 600 mM eines Ammoniumsalzes handelt;
c. Sammeln mindestens einer Fraktion aus dem Eluat von Schritt b); und
d. Analysieren der mindestens einen Fraktion durch Massenspektrometrie zur Charakterisierung der mindestens einen Ladungsvariante des Proteins von Interesse.

2. Verfahren nach Anspruch 1, ferner umfassend Deglykosylieren der Probe vor dem Laden der Probe auf die AEX-Säule.

3. Verfahren nach Anspruch 1 oder Anspruch 2, wobei es sich bei dem Ammoniumsalz-Konzentrationsgradienten um einen Elutionsgradienten im Bereich von etwa 10 mM bis etwa 300 mM handelt.

4. Verfahren nach einem der Ansprüche 1-3, wobei der zunehmende Ammoniumsalz-Konzentrationsgradient linear aufgetragen wird.

5. Verfahren nach einem der Ansprüche 1-4, ferner umfassend Überwachen der Ultraviolettextinktion des Eluats aus Schritt b) und Sammeln der mindestens einen Fraktion, die vor oder nach Elution des Proteins von Interesse eluiert wird.

6. Verfahren nach einem der Ansprüche 1-5, wobei es sich bei dem Protein von Interesse um einen monoklonalen Antikörper auf Basis von Immunoglobulin G Subklasse 4 (IgG4) handelt.

7. Verfahren nach einem der Ansprüche 1-6, wobei es sich bei dem Protein von Interesse um einen bispezifischen monoklonalen Antikörper handelt.

8. Verfahren nach einem der Ansprüche 1-7, wobei es sich bei der mindestens einen Ladungsvariante um eine Deamidierung, Glykierung, Glucuronylierung, hochmolekulare Spezies, ein C-terminales Lysin oder eine glykosylierte Spezies des Proteins von Interesse handelt.

9. Verfahren nach einem der Ansprüche 1-8, ferner umfassend Entfernen einer Fc-N-Glykosylierung aus dem Protein von Interesse vor dem Laden der Probe auf die AEX-Säule.

10. Verfahren nach einem der Ansprüche 1-9, ferner umfassend Verdauen der Probe vor dem Laden der Probe auf die AEX-Säule.

11. Verfahren nach Anspruch 10, wobei die Probe mit einem Immunglobulin G abbauenden Enzym von Streptococcus pyogenes (IdeS) oder einer Variante davon verdaut wird.

12. Verfahren nach einem der Ansprüche 1-11, wobei das Massenspektrometer unter nativen Bedingungen gefahren wird.

## Revendications

1. Procédé de caractérisation d'au moins un variant de charge d'une protéine d'intérêt ayant une valeur de point isoélectrique (pI) d'environ 6,2 à environ 7,0, le procédé comprenant :
a. chargement d'un échantillon comprenant une protéine d'intérêt et au moins un variant de charge de la protéine d'intérêt sur une colonne de chromatographie échangeuse d'anions (AEX) couplée en ligne à une spectrométrie de masse ;
b. application d'un gradient croissant de concentration de sel à la colonne AEX chargée pour obtenir un éluat, sans application d'un gradient de pH, le gradient de sel étant un gradient d'élution allant d'environ 10 mM à environ 600 mM d'un sel d'ammonium ;
c. collecte d'au moins une fraction à partir de l'éluat de l'étape b) ; et
d. analyse de l'au moins une fraction par spectrométrie de masse pour caractériser l'au moins un variant de charge de la protéine d'intérêt.

2. Procédé selon la revendication 1, comprenant en outre la déglycosylation de l'échantillon avant le chargement de l'échantillon sur la colonne AEX.

3. Procédé selon la revendication 1 ou la revendication 2, dans lequel le gradient de concentration de sel d'ammonium est un gradient d'élution allant d'environ 10 mM à environ 300 mM.

4. Procédé selon l'une quelconque des revendications 1 à 3, dans lequel le gradient croissant de concentration de sel d'ammonium est appliqué linéairement.

5. Procédé selon l'une quelconque des revendications 1 à 4, comprenant en outre la surveillance de l'absorbance des ultraviolets de l'éluat de l'étape b) et la collecte de l'au moins une fraction qui est éluée avant ou après élution de la protéine d'intérêt.

6. Procédé selon l'une quelconque des revendications 1 à 5, dans lequel la protéine d'intérêt est un anticorps monoclonal à base d'immunoglobuline G de sous-classe 4 (IgG4).

7. Procédé selon l'une quelconque des revendications 1 à 6, dans lequel la protéine d'intérêt est un anticorps monoclonal bispécifique.

8. Procédé selon l'une quelconque des revendications 1 à 7, dans lequel l'au moins un variant de charge est une désamidation, une glycation, une glucuronylation, une espèce à poids moléculaire élevé, une lysine C-terminale ou une espèce glycosylée de la protéine d'intérêt.

9. Procédé selon l'une quelconque des revendications 1 à 8, comprenant en outre l'élimination d'une N-glycosylation de Fc de la protéine d'intérêt avant le chargement de l'échantillon sur la colonne AEX.

10. Procédé selon l'une quelconque des revendications 1 à 9, comprenant en outre la digestion de l'échantillon avant le chargement de l'échantillon sur la colonne AEX.

11. Procédé selon la revendication 10, dans lequel l'échantillon est digéré avec une enzyme, de *Streptococcus pyogenes*, dégradant l'immunoglobuline G (IdeS) ou un variant correspondant.

12. Procédé selon l'une quelconque des revendications 1 à 11, dans lequel le spectromètre de masse est exécuté dans des conditions natives.
